# EUROPEAN PATENT SPECIFICATION

(11) **EP 4 486 419 B1**
(45) Date of publication and mention of the grant of the patent: **04.11.2026**
(21) Application number: 23707723.5
(22) Date of filing: 27.02.2023
(51) Int. Cl.: A61M 15/00, A61M 15/08

(54) **IMPROVEMENTS RELATING TO NASAL PIECES**
VERBESSERUNGEN AN NASENSTÜCKEN
AMÉLIORATIONS APPORTÉES À DES PIÈCES NASALES

(30) Priority: 02.03.2022 GB 202202890
(43) Date of publication of application: 08.01.2025
(73) Proprietor: Bespak Europe Limited, North Lynn Industrial Estate King's Lynn Norfolk PE30 2JJ (GB)
(72) Inventor: RODRIGUEZ, Liset, Norfolk PE30 2JJ (GB)
(74) Representative: Boult Wade Tennant LLP
(86) International application number: PCT/EP2023/054804
(87) International publication number: WO 2023/165922

(56) References cited:
- EP-A1- 2 174 720
- EP-A1- 3 162 402
- EP-B1- 2 844 315
- WO-A1-2012/063124
- WO-A1-2021/066195
- GB-A- 191 306 078
- RU-C1- 2 605 794
- US-A1- 2005 258 191
- US-A1- 2019 151 576
- US-A1- 2020 171 252

## Description

The present disclosure relates to nasal pieces. In addition, it relates to nasal sprays, nasal cannulas, nebulizers or nasal dilators comprising a nasal piece, as well as use of a nasal piece and a method of manufacturing a nasal piece.

### Background to the Disclosure

A nasal piece is an item or component of a device designed to engage in a nostril of a user. A nasal piece may be intended to permit the delivery of a substance into the nasal passages, for example when provided as part of a nasal spray, nasal cannula or nebulizer. A nasal piece may also (or in addition) be intended to open the nostril of a user, for example when provided as, or as part of, a nasal dilator.

The sizing of a nasal piece relative to the size of a user's nostril may, in many cases, be important to maximise the benefits of the item or device. For example, in cases where the nasal piece is used to deliver a substance into the nasal passages, the efficiency of the delivery of the substance may be affected by factors including the degree of sealing between the nasal piece and the nostril and the location of an outlet orifice of the nasal piece vis-à-vis the users' nasal cavity. In cases where the nasal piece is used to dilate the nostril, the efficiency and comfort of the dilation will be affected by factors including the size of the nasal piece vis-à-vis the user's nostril size.

Typically, nasal pieces are provided in a single size only for each device for reasons such as manufacturing economy. This may result in a decreased efficiency of substance delivery or nostril dilation for many users considering the wide range of nostril sizes of users.

WO2012063124 discloses an apparatus comprising: a fluid discharge orifice defined in a distal end of a nasal stopper, the distal end being sufficiently small in cross-section as to permit entrance of the discharge orifice into a nostril opening of a human child, a proximal portion of the nasal stopper being configured to resist over- insertion of the discharge orifice into the opening; an atomizer comprising the discharge orifice, the discharge orifice being disposed in a wetted fluid path to conduct fluid from a turbine chamber; and a stem structured to provide a lumen for communication of treatment fluid to the turbine chamber for discharge of the fluid substantially as a mist from the discharge opening, the stem extending in a length direction between a proximal end and a distal end, wherein: thread structure carried at the proximal end of the stem is configured to couple with a lure- locking portion of a syringe.

WO2021066195 discloses nasal spray injection nozzle has: a distal end portion having a nozzle injection hole; and a nose rest that can be brought into contact with a portion around a nostril.

GB191306078 discloses an appliance for nasal inhalation comprising a container having a perforated wall tapered to a point adapted to enter the nostril, said wall having perforations arranged close to the point and remote from the point respectively, in combination with a removable cap which, fitted on said container, covers all said perforations and is screwed to the wall of the container at a part between the large end of the latter and the perforations remote from the point.

EP3162402 discloses a rhinal spray nozzle used for a medical syringe having a tip opening in fluid communication with a syringe barrel for storing a pharmaceutical formulation. The rhinal spray nozzle comprises a hollow nozzle body having a tip portion defining a nozzle orifice thereon, a solid packing rod arranged within the nozzle body, and a nozzle chamber defined between the packing rod and the nozzle body to allow a fluid communication between the tip opening and the nozzle orifice, wherein the formulation comprises the gel material containing viscosity modification agent and carboxy vinyl polymer of which viscosity is modified by applying an exogenous shear force, and wherein the nozzle orifice has a diameter in a range between 0.25 mm and 0.30 mm.

US2020171252 discloses a discharge head for the nasal application of pharmaceutical liquid from a pressure reservoir which has an outlet valve with a valve connector, to which force can be applied counter to a spring force in order to open the outlet valve. The discharge head has a nasal applicator which extends outward from an actuating surface and at the end of which a discharge opening is provided. The discharge opening is connected in a fluid-communicating manner to a hollow tube through an applicator channel of the nasal applicator, for connection to the pressure reservoir. The nasal applicator has an inner component connected integrally to the actuating surface and an outer component separate from the inner component and attached to the inner component in a surrounding manner. The discharge opening is provided in the outer component, and the outer component and the inner component together bound the applicator channel.

US2005258191 discloses a fluid dispenser having a housing and a pump action fluid discharge device. The pump action fluid discharge device is arranged to be actuated by one or more levers which are pivotally supported within the housing. When the or each lever is rotated about its lower end the fluid discharge device is urged towards a nozzle causing a single dose of fluid to be dispensed from the nozzle.

EP2844315 discloses a kit for performing nasal irrigation by spraying or dropping a fluid into a nostril, comprising a container, a cap, first means of communication, second means of communication, a first draft tube, a dispenser and a flexible connection tube.

EP2174720 discloses an application device with blocking means.

RU2605794 discloses a replaceable filter. The filter is made in form of tampon, body of which is shaped as truncated cone with circular notches for retention in nasal cavity of user.

### Summary of the Disclosure

The invention is defined in the appended claims.

Advantageously, the nasal piece of the present disclosure may provide improved sealing in a wide range of sizes of nostril. The provision of separate first and second flared sections may permit a single nasal piece to be suitable for use by both adult and paediatric users.

For example, the first flared section may be configured for use by paediatric users or a sub-range of paediatric users. In some examples the first flared section may be configured for use by users aged 0-5 years.

For example, the second flared section may be configured for use by adult users (and optionally also older paediatric users). In some examples the second flared section may be configured for use by users aged 5 years and above, optionally 18 years and above.

Configuring the first abutment zone to have a greater maximum flare angle than the second abutment zone may be advantageous in better configuring the nasal piece for both paediatric and adult users. In particular, the variation in nostril diameter for paediatric users, especially those under 7 years of age, is relatively large. For this reason it has been found that providing a relatively large maximum flare angle for the first abutment zone, and hence a steeper slope to the nasal piece in this area, is beneficial in accommodating a large variation in nostril diameter without leading to over-insertion.

Advantageously, the nasal piece of the present disclosure may provide a single nasal piece that may be used by many, and potentially all, ages of user. The nasal piece may also at the same time increase the comfort and efficiency of the nasal piece by better configuring the location of abutment of the nasal piece with the nostril for a range of users having a range of nostril sizes. In addition, the nasal piece may be better suited for use by both male and female users who may, typically, have differing ranges of nostril sizes.

Advantageously, the nasal piece of the present disclosure may reduce the cost and complexity of manufacture of devices by avoiding (or at least reducing) the need to provide a range of separate nasal pieces of differing sizes for a single device.

The first abutment zone may comprise a whole or a part of the first flared section. The second abutment zone may comprise a whole or a part of the second flared section.

The first flared section and/or the second flared section of the nasal piece may have circular symmetry about the longitudinal axis of the nasal piece. Alternatively, the first flared section and/or the second flared section of the nasal piece may not have circular symmetry about the longitudinal axis of the nasal piece, e.g. an external diameter of the first flared section and/or the second flared section may differ front-to-back from side-side.

The maximum flare angle of the first abutment zone may be 35° to 65°, preferably 40° to 60°, more preferably 58° and/or the maximum flare angle of the second abutment zone may be 10° to 30°, preferably 15° to 25°, more preferably 18°.

In some examples, a flare angle of the first abutment zone may be constant along a longitudinal length of the first abutment zone. For example, the first abutment zone may be provided by a frusto-conical section of the body. The constant flare angle of the first abutment zone may be, for example, 35° to 65°, preferably 40° to 60°, more preferably 58°.

In alternative examples, a flare angle of the first abutment zone may vary along a longitudinal length of the first abutment zone. For example, the first abutment zone may be provided by a concavely-shaped (as viewed from an exterior of the nasal piece) section of the body or alternatively a series of frusto-conical sections. The flare angle may vary, for example, from a minimum flare angle of 15° to 20° to the maximum flare angle of 35° to 65°, preferably 40° to 60°, more preferably 58°. In examples where the first abutment zone is concavely-shaped, the first abutment zone may have a constant or variable radius of curvature along its longitudinal length.

In some examples, a flare angle of the second abutment zone may be constant along a longitudinal length of the second abutment zone. For example, the second abutment zone may be provided by a frusto-conical section of the body. The constant flare angle of the second abutment zone may be, for example, 10° to 30°, preferably 15° to 25°, more preferably 18°.

In alternative examples, a flare angle of the second abutment zone may vary along a longitudinal length of the second abutment zone. For example, the second abutment zone may be provided by a concavely-shaped (as viewed from an exterior of the nasal piece) section of the body or alternatively a series of frusto-conical sections. The flare angle may, for example, vary from a minimum flare angle of 5° to 10° to the maximum flare angle of 10° to 30°, preferably 15° to 25°, more preferably 18°. In examples where the second abutment zone is concavely-shaped, the second abutment zone may have a constant or variable radius of curvature along its longitudinal length.

Preferably, a longitudinal length of the first abutment zone and/or the first flared section is shorter than a longitudinal length of the second abutment zone and/or the second flared section. Beneficially, this may help to prevent over-insertion of the tip of the nasal piece into the nostrils of paediatric users, especially those under 7 years of age.

In some examples the first abutment zone extends at least between locations that are 5.0 mm to 7.0 mm longitudinally from the tip. This has been found to be a beneficial location for engaging the nostril of paediatric users and providing good positioning of the tip of the nasal piece vis-à-vis the nasal valve of the nasal cavity.

In some examples the second abutment zone extends at least between locations that are 12.0 mm to 15.0 mm longitudinally from the tip; optionally at least between locations that are 12.0 mm to 20.0 mm longitudinally from the tip; optionally at least between locations that are 10.0 mm to 20.0 mm longitudinally from the tip. This has been found to be a beneficial location for engaging the nostril of adult users providing and good positioning of the tip of the nasal piece vis-à-vis the nasal valve of the nasal cavity.

Preferably, the second flared section is wider than the first flared section.

In some examples an external diameter of the first abutment zone at its end nearest the tip may have a diameter of 6.60 to 7.50 mm, optionally of 6.90 to 7.20 mm, optionally of 7.09 mm; and/or an external diameter of the first abutment zone at its end furthest from the tip may have a diameter of 9.50 to 11.00 mm, optionally of 9.75 to 10.50 mm, optionally of 10.43 mm.

In some examples an external diameter of the second abutment zone at its end nearest the tip has a diameter of 10.50 to 11.50 mm, optionally of 10.70 to 11.00 mm, optionally of 10.70 to 10.90 mm, optionally of 10.85 mm or 10.96 mm; and/or an external diameter of the second abutment zone at its end furthest from the tip has a diameter of 11.00 to 20.00 mm, optionally 11.00 to 16.00 mm, optionally 11.00 to 15.00 mm, optionally of 11.75 to 15.00 mm, optionally of 14.00 to 15.00 mm, optionally of 11.75 mm or 14.75 mm.

The body comprises a shoulder or convex section that separates at least a portion of the first abutment zone from at least a portion of the second abutment zone. In some examples the shoulder or convex section may overlap partially with the first abutment zone and/or the second abutment zone. In some examples the shoulder or convex section may overlap wholly or partially with the first flared section and/or the second flared section.

Provision of the shoulder or convex section of the nasal piece may beneficially allow for a more compact nasal piece, in particular in shortening the longitudinal length of the nasal piece. In addition, the provision of the shoulder or convex section permits the wider, second abutment zone to be positioned closer to the tip of the nasal piece than would be the case with a nasal piece having a single taper. This may help to prevent over-insertion of the tip of the nasal piece into the nostrils of users.

In some examples the shoulder or convex section of the body is located between 5.0 mm and 10.0 mm longitudinally from the tip.

The first flared section comprises a concave section and the second section comprises a concave section. Either or both of the first abutment zone and the second abutment zone may be in the form of one or more frusto-conical sections. Either or both of the first abutment zone and the second abutment zone may be in the form of one or more concavely-shaped sections. Either or both of the first abutment zone and the second abutment zone may be in the form of one or more convexly-shaped sections. Frusto-conical section(s) and concavely-shaped section(s) and convexly-shaped section(s) may be combined in the same nasal piece.

In some examples a longitudinal axis of the first flared section is coincident with a longitudinal axis of the second flared section.

The body comprises a tip section that comprises the tip of the body and extends from the tip to the first flared section, wherein the tip section is convex.

In some examples, for example where the nasal piece is for use as part of a nasal spray, the body further comprises one or more shoulders for engagement by one or more fingers, the one or more shoulders being located further from the tip than the second flared section.

Another embodiment of the present disclosure provides a nasal spray, nasal cannula, nebulizer or nasal dilator comprising a nasal piece as described in the above embodiment. In the case of a nasal spray the device may, for example, be of a type used for nasal inhalation of a medicament.

Another embodiment of the present disclosure is use of a nasal piece of the first embodiment above, wherein in a first mode of use the nasal piece is inserted into a first nostril having a first range of sizes such that the first nostril is abutted against the first abutment zone of the first flared section; and in a second mode of use the nasal piece is inserted into a second nostril having a second range of sizes such that the second nostril is abutted against the second abutment zone of the second flared section.

The first nostril may be a nostril of a paediatric user and the second nostril may be a nostril of an adult user.

Another embodiment of the present disclosure provides a method of manufacturing a nasal piece of the first embodiment above, the method comprising forming the body of the nasal piece to have the tip, the first flared section that widens in a longitudinal direction away from the tip and the second flared section that also widens in the longitudinal direction away from the tip, wherein the first flared section is more proximate to the tip than the second flared section, and the first flared section defines the first abutment zone for engaging in the first nostril having the first range of sizes, and the second flared section defines the second abutment zone for engaging in the second nostril having the second range of sizes, wherein the first abutment zone has a maximum flare angle that is greater than a maximum flare angle of the second abutment zone.

In some examples the nasal piece is formed as a single piece; and optionally formed by moulding, preferably injection moulding.

### Brief Description of the Drawings

One or more embodiments of the disclosure will now be described, by way of example only, with reference to the accompanying drawings in which:
Figure 1 is a front elevation view of a nasal piece according to the present disclosure;
Figure 2 is a plan view from above of the nasal piece of Figure 1;
Figure 3 is a perspective view from above of the nasal piece of Figure 1;
Figure 4 is a side elevation view of the nasal piece of Figure 1 depicting flare angles of the nasal piece;
Figures 5 to 7 are side elevation views of the nasal piece of Figure 1 depicting dimensions of the nasal piece;
Figure 8A is a partial front elevation view of a nasal piece according to the present disclosure comprising concavely-shaped abutment zones;
Figure 8B is a partial front elevation view of a nasal piece according to the present disclosure comprising frusto-conical abutment zones; and Figure 9 is a front elevation view of a nasal spray comprising the nasal piece of Figure 1.

### Detailed Description

Unless defined otherwise, all technical and scientific terms used in this specification have the same meaning as is commonly understood by the reader skilled in the art to which the claimed subject matter belongs. It is to be understood that the foregoing summary of the disclosure and the following examples are exemplary and explanatory only and are not restrictive of any subject matter claimed.

The following description is directed to embodiments of the disclosure. The description of the embodiments is not meant to include all the possible embodiments of the disclosure that are claimed in the appended claims. Many modifications, improvements and equivalents which are not explicitly recited in the following embodiments may fall within the scope of the appended claims. Features described as part of one embodiment may be combined with features of one or more other embodiments unless the context clearly requires otherwise.

In this specification, the use of the singular includes the plural unless the context clearly dictates otherwise. In this application, the use of "and/or" means "and" and "or" unless stated otherwise.

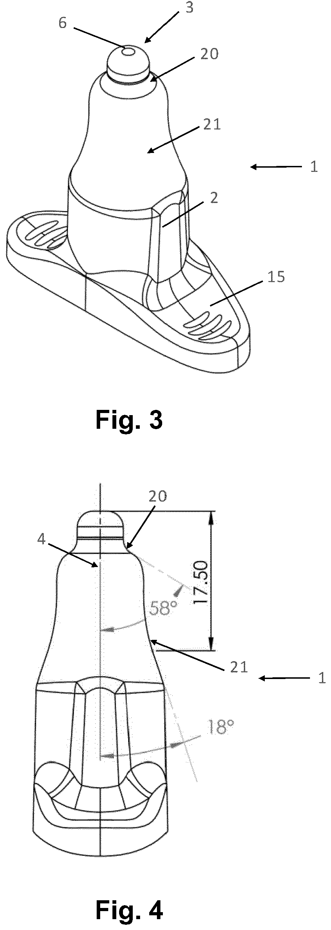

In this specification, the term "flare angle" means for a point on the external surface of the nasal piece, the angle between the longitudinal axis of the nasal piece and the tangent to the external surface at that point - as shown, by way of example, in Figure 4.

The present disclosure relates generally to nasal pieces. In addition, it relates to nasal sprays, nasal cannulas, nebulizers or nasal dilators comprising a nasal piece, as well as use of a nasal piece and a method of manufacturing a nasal piece.

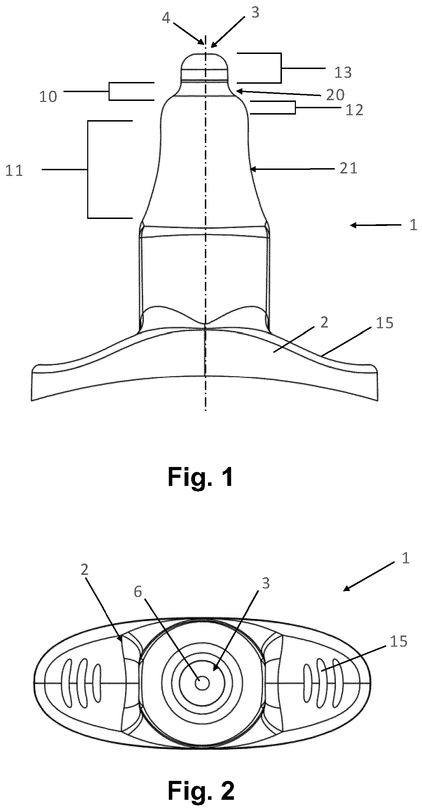

Figures 1 to 7 show a nasal piece 1 comprising a body 2 having a tip 3 for insertion into a nostril of a user. As shown in Figure 1, the body 2 comprises a first flared section 10 that widens in a longitudinal direction away from the tip 3 and a second flared section 11 that also widens in the longitudinal direction away from the tip 3. The first flared section 10 is more proximate to the tip 3 than the second flared section 11.

The first flared section 10 defines a first abutment zone 20 for engaging in a first nostril having a first range of sizes.

The second flared section 11 define a second abutment zone 21 for engaging in a second nostril having a second range of sizes.

The first abutment zone 20 has a maximum flare angle that is greater than a maximum flare angle of the second abutment zone 21.

The maximum flare angle of the first abutment zone 20 may be 35° to 65°, preferably 40° to 60°. More preferably, as shown in Figure 4, the maximum flare angle of the first abutment zone 20 may be 58°.

The maximum flare angle of the second abutment zone 21 may be 10° to 30°, preferably 15° to 25°. More preferably, as shown in Figure 4, the maximum flare angle of the second abutment zone 21 may be 18°.

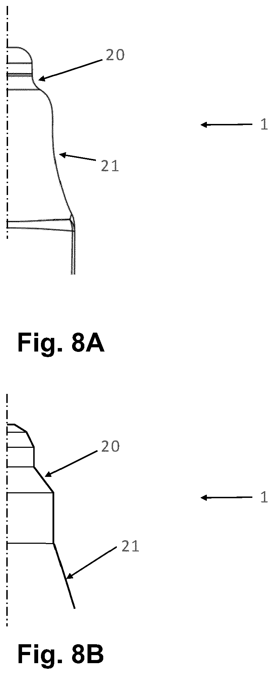

The flare angle of the first abutment zone 20 may be constant along a longitudinal length of the first abutment zone 20. This may be the case, for example, where the first flared section 10 has a frusto-conical form as shown, by way of example, in Figure 8B. For example, the constant flare angle of the first abutment zone 20 may be 35° to 65°, preferably 40° to 60°, more preferably 58°.

Alternatively, the flare angle of the first abutment zone 20 may vary along the longitudinal length of the first abutment zone 20. This may be the case, for example, where the first flared section 10 has a concavely-shaped form as shown, by way of example, in Figure 8A. For example, the flare angle may vary from a minimum flare angle of 15° to 20° to the maximum flare angle of 35° to 65°, preferably 40° to 60°, more preferably 58°.

Similarly, the flare angle of the second abutment zone 21 may be constant along the longitudinal length of the second abutment zone 21. This may be the case, for example, where the second flared section 11 has a frusto-conical form as shown, by way of example, in Figure 8B. For example, the constant flare angle of the second abutment zone 21 may be 10° to 30°, preferably 15° to 25°, more preferably 18°.

Alternatively, the flare angle of the second abutment zone 21 may vary along the longitudinal length of the second abutment zone 21. This may be the case, for example, where the second flared section 11 has a concavely-shaped form as shown, by way of example, in Figure 8A. For example, the flare angle may vary from a minimum flare angle of 5° to 10° to the maximum flare angle of 10° to 30°, preferably 15° to 25°, more preferably 18°.

The longitudinal length of the first abutment zone 20 and/or the first flared section 10 may be shorter than the longitudinal length of the second abutment zone 21 and/or the second flared section 11.

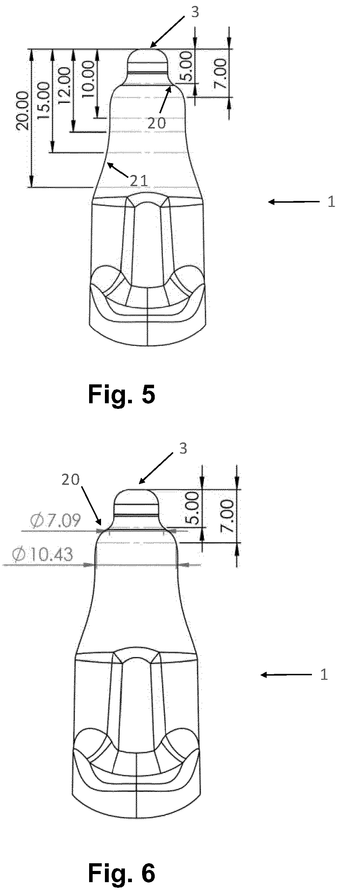

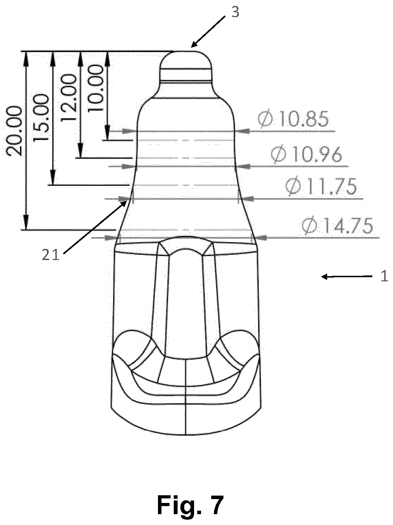

The first abutment zone 20 may extend at least between locations that are 5.0 mm to 7.0 mm longitudinally from the tip 3, as shown by way of example in Figures 5 and 6. The second abutment zone 21 may extend at least between locations that are 12.0 mm to 15.0 mm longitudinally from the tip 3; optionally at least between locations that are 12.0 mm to 20.0 mm longitudinally from the tip 3; optionally at least between locations that are 10.0 mm to 20.0 mm longitudinally from the tip 3, as shown by way of example in Figures 5 and 7.

The second flared section 11 may be wider than the first flared section 10.

An external diameter of the first abutment zone 20 at its end nearest the tip may have a diameter of 6.60 to 7.50 mm, optionally of 6.90 to 7.20 mm, optionally of 7.09 mm. The measurement may be, for example, at 5.00 mm longitudinally from the tip 3 as shown in the example of Figure 6. An external diameter of the first abutment zone 20 at its end furthest from the tip 3 may have a diameter of 9.50 to 11.00 mm, optionally of 9.75 to 10.50 mm, optionally of 10.43 mm. The measurement may be, for example, at 7.00 mm longitudinally from the tip 3 as shown in the example of Figure 6.

An external diameter of the second abutment zone 21 at its end nearest the tip may have a diameter of 10.50 to 11.50 mm, optionally of 10.70 to 11.00 mm, optionally of 10.70 to 10.90 mm, optionally of 10.85 mm or 10.96 mm. The measurement may be, for example, at 7.00 mm or 10.00 mm longitudinally from the tip 3 as shown in the example of Figure 7. An external diameter of the second abutment zone 21 at its end furthest from the tip 3 may have a diameter of 11.00 to 20.00 mm, optionally 11.00 to 16.00 mm, optionally 11.00 to 15.00 mm, optionally of 11.75 to 15.00 mm, optionally of 14.00 to 15.00 mm, optionally of 11.75 mm or 14.75 mm. The measurement may be, for example, at 15.00 mm or 20.00 mm longitudinally from the tip 3 as shown in the example of Figure 7.

The body 2 comprises a shoulder or convex section 12 that separates at least a portion of the first abutment zone 20 from at least a portion of the second abutment zone 21. The shoulder or convex section 12 of the body 2 may be located between 5.0 mm and 10.0 mm longitudinally from the tip 3. The shoulder or convex section 12 may overlap partially with the first abutment zone 20 and/or the second abutment zone 21. Additionally or alternatively the shoulder or convex section 12 may overlap wholly or partially with the first flared section 10 and/or the second flared section 11.

The first flared section 10 comprises a concave section and the second flared section 11 comprises a concave section.

The longitudinal axis of the first flared section 10 may be coincident with the longitudinal axis of the second flared section 11, as in the example of Figure 1 where the longitudinal axis is labelled 4.

The body 2 comprises a tip section 13 that comprises the tip 3 of the body 2 and extends from the tip 3 to the first flared section 10, wherein the tip section 13 is convex as shown in Figures 1 and 3. An outlet orifice 6 may be provided in the tip section 13, optionally at the apex of the tip 3, as shown in Figures 2 and 3.

The body 2 may further comprise, as shown in Figures 1 and 3, one or more shoulders 15 for engagement by one or more fingers, the one or more shoulders 15 being located further from the tip 3 than the second flared section 11.

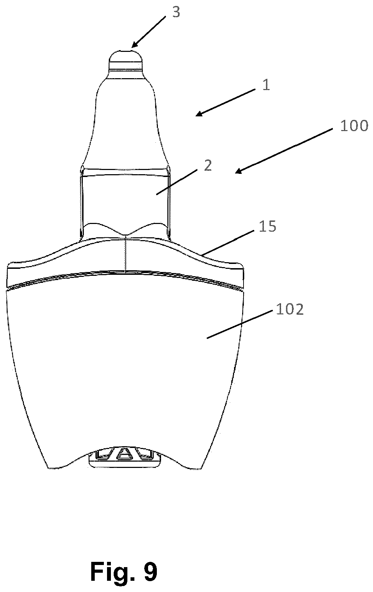

A nasal spray, nasal cannula, nebulizer or nasal dilator may be provided that comprises a nasal piece 1 as described above. An example of a nasal spray 100 is shown in Figure 9, wherein the nasal piece 1 is mounted on top of a body 102 of the nasal spray 100. In use, a source of product, for example a medicament to be delivered nasally, may be housed within the body 102.

The nasal piece 1 of the present disclosure may be used in a first mode of use wherein the nasal piece 1 is inserted into a first nostril having a first range of sizes such that the first nostril is abutted against the first abutment zone 20 of the first flared section 10 and in a second mode of use wherein the nasal piece 1 is inserted into a second nostril having a second range of sizes such that the second nostril is abutted against the second abutment zone 21 of the second flared section 11. For example, the first nostril may be a nostril of a paediatric user and the second nostril may be a nostril of an adult user.

The nasal piece 1 of the present disclosure may be manufactured so as to be formed as a single piece. The nasal piece 1 may be, for example, formed by moulding, preferably injection moulding.

### Industrial Applicability

The present disclosure finds application in the provision of nasal pieces for devices, for example nasal sprays, nasal cannulas, and nebulizers or nasal dilators comprising a nasal piece.

It is to be understood that at least some of the figures and descriptions of the disclosure have been simplified to focus on elements that are relevant for a clear understanding of the disclosure, while eliminating, for purposes of clarity, other elements that the reader skilled in the art will appreciate may also be required. Because such elements are well known to the reader skilled in the art, and because they do not necessarily facilitate a better understanding of the disclosure, a description of such elements is not provided herein.

## Claims

1. A nasal piece (1) comprising a body (2) having a tip (3) for insertion into a range of sizes of nostril, the body (2) comprising:
a first flared section (10) that widens in a longitudinal direction away from the tip (3);
a second flared section (11) that also widens in the longitudinal direction away from the tip (3);
the first flared section (10) being more proximate to the tip (3) than the second flared section (11);
the first flared section (10) defining a first abutment zone (20) for engaging in a first nostril having a first range of sizes;
the second flared section (11) defining a second abutment zone (21) for engaging in a second nostril having a second range of sizes;
wherein the first abutment zone (20) has a maximum flare angle that is greater than a maximum flare angle of the second abutment zone (21);
wherein the body (2) comprises a shoulder or convex section (12) that separates at least a portion of the first abutment zone (20) from at least a portion of the second abutment zone (21);
wherein the first flared section (10) comprises a concave section and the second flared section (11) comprises a concave section; and
wherein the body (2) further comprises a tip section (13) that comprises the tip (3) of the body (2) and extends from the tip (3) to the first flared section (10), wherein the tip section (13) is convex.

2. A nasal piece as claimed in claim 1, wherein the maximum flare angle of the first abutment zone (20) is 35° to 65°, preferably 40° to 60°, more preferably 58° and/or the maximum flare angle of the second abutment zone (21) is 10° to 30°, preferably 15° to 25°, more preferably 18°.

3. A nasal piece as claimed in claim 1 or claim 2, wherein either:
a flare angle of the first abutment zone (20) is constant along a longitudinal length of the first abutment zone (20); and optionally wherein the flare angle of the first abutment zone (20) is 35° to 65°, preferably 40° to 60°, more preferably 58°;
or
a flare angle of the first abutment zone (20) varies along a longitudinal length of the first abutment zone (20); and optionally wherein the flare angle varies from a minimum flare angle of 15° to 20° to the maximum flare angle of 35° to 65°, preferably 40° to 60°, more preferably 58°.

4. A nasal piece as claimed in any preceding claim, wherein either:
a flare angle of the second abutment zone (21) is constant along a longitudinal length of the second abutment zone (21); and optionally wherein the flare angle of the second abutment zone (21) is 10° to 30°, preferably 15° to 25°, more preferably 18°;
or
a flare angle of the second abutment zone (21) varies along a longitudinal length of the second abutment zone (21); and optionally wherein the flare angle varies from a minimum flare angle of 5° to 10° to the maximum flare angle of 10° to 30°, preferably 15° to 25°, more preferably 18°.

5. A nasal piece as claimed in any preceding claim, wherein a longitudinal length of the first abutment zone (20) and/or the first flared section (10) is shorter than a longitudinal length of the second abutment zone (21) and/or the second flared section (11).

6. A nasal piece as claimed in any preceding claim, wherein:
the first abutment zone (20) extends at least between locations that are 5.0 mm to 7.0 mm longitudinally from the tip (3); and/or
the second abutment zone (21) extends at least between locations that are 12.0 mm to 15.0 mm longitudinally from the tip (3); optionally at least between locations that are 12.0 mm to 20.0 mm longitudinally from the tip (3); optionally at least between locations that are 10.0 mm to 20.0 mm longitudinally from the tip (3).

7. A nasal piece as claimed in any preceding claim, wherein the second flared section (11) is wider than the first flared section (10).

8. A nasal piece as claimed in any preceding claim, wherein:
an external diameter of the first abutment zone (20) at its end nearest the tip (3) has a diameter of 6.60 to 7.50 mm, optionally of 6.90 to 7.20 mm, optionally of 7.09 mm; and/or an external diameter of the first abutment zone (20) at its end furthest from the tip (3) has a diameter of 9.50 to 11.00 mm, optionally of 9.75 to 10.50 mm, optionally of 10.43 mm; and/or
an external diameter of the second abutment zone (21) at its end nearest the tip (3) has a diameter of 10.50 to 11.50 mm, optionally of 10.70 to 11.00 mm, optionally of 10.70 to 10.90 mm, optionally of 10.85 mm or 10.96 mm; and/or an external diameter of the second abutment zone (21) at its end furthest from the tip (3) has a diameter of 11.00 to 20.00 mm, optionally 11.00 to 16.00 mm, optionally 11.00 to 15.00 mm, optionally of 11.75 to 15.00 mm, optionally of 14.00 to 15.00 mm, optionally of 11.75 mm or 14.75 mm.

9. A nasal piece as claimed in any preceding claim, wherein the shoulder or convex section (12) of the body (2) is located between 5.0 mm and 10.0 mm longitudinally from the tip (3).

10. A nasal piece as claimed in any preceding claim, wherein a longitudinal axis of the first flared section (10) is coincident with a longitudinal axis of the second flared section (11).

11. A nasal piece as claimed in any preceding claim, wherein the body (2) further comprises one or more shoulders (15) for engagement by one or more fingers, the one or more shoulders (15) being located further from the tip (3) than the second flared section (11).

12. A nasal spray, nasal cannula, nebulizer or nasal dilator comprising a nasal piece as claimed in any preceding claim.

13. Use of a nasal piece as claimed in any one of claims 1 to 11, wherein in a first mode of use the nasal piece is inserted into a first nostril having a first range of sizes such that the first nostril is abutted against the first abutment zone (20) of the first flared section (10); and in a second mode of use the nasal piece is inserted into a second nostril having a second range of sizes such that the second nostril is abutted against the second abutment zone (21) of the second flared section (11);
wherein optionally the first nostril is a nostril of a paediatric user and the second nostril is a nostril of an adult user.

14. A method of manufacturing a nasal piece as claimed in any one of claims 1 to 11, comprising forming the body (2) of the nasal piece to have the tip (3), the first flared section (10) that widens in a longitudinal direction away from the tip (3) and the second flared section (11) that also widens in the longitudinal direction away from the tip (3), wherein the first flared section (10) is more proximate to the tip than the second flared section (11), and the first flared section (10) defines the first abutment zone (20) for engaging in the first nostril having the first range of sizes, and the second flared section (11) defines the second abutment zone (21) for engaging in the second nostril having the second range of sizes, wherein the first abutment zone (20) has a maximum flare angle that is greater than a maximum flare angle of the second abutment zone (21);
wherein optionally the nasal piece is formed as a single piece; and optionally formed by moulding, preferably injection moulding.

## Patentansprüche

1. Nasenstück (1), umfassend einen Körper (2), der eine Spitze (3) zur Einführung in einen Größenbereich von Nasenlöcher aufweist, wobei der Körper (2) Folgendes umfasst:
einen ersten aufgeweiteten Abschnitt (10), der sich in einer Längsrichtung von der Spitze (3) weg verbreitert;
einen zweiten aufgeweiteten Abschnitt (11), der sich ebenfalls in der Längsrichtung von der Spitze (3) weg verbreitert;
wobei der erste aufgeweitete Abschnitt (10) näher an der Spitze (3) vorliegt als der zweite aufgeweitete Abschnitt (11);
wobei der erste aufgeweitete Abschnitt (10) eine erste Anstoßzone (20) zum Eingreifen in ein erstes Nasenloch mit einem ersten Größenbereich definiert;
wobei der zweite aufgeweitete Abschnitt (11) eine zweite Anstoßzone (21) zum Eingreifen in ein zweites Nasenloch mit einem zweiten Größenbereich definiert;
wobei die erste Anstoßzone (20) einen maximalen Aufweitungswinkel aufweist, der größer ist als ein maximaler Aufweitungswinkel der zweiten Anstoßzone (21);
wobei der Körper (2) einen Schulter- oder konvexen Abschnitt (12) umfasst, der mindestens einen Teil der ersten Anstoßzone (20) von mindestens einem Teil der zweiten Anstoßzone (21) trennt;
wobei der erste aufgeweitete Abschnitt (10) einen konkaven Abschnitt umfasst und der zweite aufgeweitete Abschnitt (11) einen konkaven Abschnitt umfasst; und
wobei der Körper (2) weiter einen Spitzenabschnitt (13) umfasst, der die Spitze (3) des Körpers (2) umfasst und sich von der Spitze (3) bis zu dem ersten aufgeweiteten Abschnitt (10) erstreckt, wobei der Spitzenabschnitt (13) konvex ist.

2. Nasenstück nach Anspruch 1, wobei der maximale Aufweitungswinkel der ersten Anstoßzone (20) 35° bis 65°, vorzugsweise 40° bis 60°, bevorzugter 58° beträgt und/oder der maximale Aufweitungswinkel der zweiten Anstoßzone (21) 10° bis 30°, vorzugsweise 15° bis 25°, bevorzugter 18° beträgt.

3. Nasenstück nach Anspruch 1 oder Anspruch 2, wobei entweder:
ein Aufweitungswinkel der ersten Anstoßzone (20) entlang einer längsgerichteten Länge der ersten Anstoßzone (20) konstant ist; und wobei der Aufweitungswinkel der ersten Anstoßzone (20) optional 35° bis 65°, vorzugsweise 40° bis 60°, bevorzugter 58° beträgt;
oder
der Aufweitungswinkel der ersten Anstoßzone (20) entlang einer längsgerichteten Länge der ersten Anstoßzone (20) variiert; und wobei der Aufweitungswinkel optional von einem minimalen Aufweitungswinkel von 15° bis 20° bis zu dem maximalen Aufweitungswinkel von 35° bis 65°, vorzugsweise 40° bis 60°, bevorzugter 58° variiert.

4. Nasenstück nach einem vorstehenden Anspruch, wobei entweder:
ein Aufweitungswinkel der zweiten Anstoßzone (21) entlang einer längsgerichteten Länge der zweiten Anstoßzone (21) konstant ist; und wobei der Aufweitungswinkel der zweiten Anstoßzone (21) optional 10° bis 30°, vorzugsweise 15° bis 25°, bevorzugter 18° beträgt;
oder
ein Aufweitungswinkel der zweiten Anstoßzone (21) entlang einer längsgerichteten Länge der zweiten Anstoßzone (21) variiert; und wobei der Aufweitungswinkel optional von einem minimalen Aufweitungswinkel von 5° bis 10° bis zu dem maximalen Aufweitungswinkel von 10° bis 30°, vorzugsweise 15° bis 25°, bevorzugter 18° variiert.

5. Nasenstück nach einem vorstehenden Anspruch, wobei die längsgerichtete Länge der ersten Anstoßzone (20) und/oder der erste aufgeweitete Abschnitt (10) kürzer ist als eine längsgerichtete Länge der zweiten Anstoßzone (21) und/oder des zweiten aufgeweiteten Abschnitts (11).

6. Nasenstück nach einem vorstehenden Anspruch, wobei:
sich die erste Anstoßzone (20) mindestens zwischen Stellen erstreckt, die längsgerichtet 5,0 mm bis 7,0 mm von der Spitze (3) vorliegen; und/oder
sich die zweite Anstoßzone (21) mindestens zwischen Stellen, die längsgerichtet 12,0 mm bis 15,0 mm von der Spitze (3) vorliegen; optional mindestens zwischen Stellen, die längsgerichtet 12,0 mm bis 20,0 mm von der Spitze (3) vorliegen; optional mindestens zwischen Stellen, die längsgerichtet 10,0 mm bis 20,0 mm von der Spitze (3) vorliegen erstreckt.

7. Nasenstück nach einem vorstehenden Anspruch, wobei der zweite aufgeweitete Abschnitt (11) breiter ist als der erste aufgeweitete Abschnitt (10).

8. Nasenstück nach einem vorstehenden Anspruch, wobei:
ein Außendurchmesser der ersten Anstoßzone (20) an ihrem der Spitze (3) nächstgelegenen Ende einen Durchmesser von 6,60 bis 7,50 mm, optional von 6,90 bis 7,20 mm, optional von 7,09 mm aufweist; und/oder ein Außendurchmesser der ersten Anstoßzone (20) an ihrem von der Spitze (3) entferntesten Ende einen Durchmesser von 9,50 bis 11,00 mm, optional von 9,75 bis 10,50 mm, optional von 10,43 mm aufweist; und/oder
ein Außendurchmesser der zweiten Anstoßzone (21) an ihrem der Spitze (3) nächstgelegenen Ende einen Durchmesser von 10,50 bis 11,50 mm, optional von 10,70 bis 11,00 mm, optional von 10,70 bis 10,90 mm, optional von 10,85 mm oder 10,96 mm aufweist; und/oder ein Außendurchmesser der zweiten Anstoßzone (21) an ihrem von der Spitze (3) entferntesten Ende einen Durchmesser von 11,00 bis 20,00 mm, optional 11,00 bis 16,00 mm, optional 11,00 bis 15,00 mm, optional von 11,75 bis 15,00 mm, optional von 14,00 bis 15,00 mm, optional von 11,75 mm oder 14,75 mm aufweist.

9. Nasenstück nach einem vorstehenden Anspruch, wobei sich der Schulter- oder konvexe Abschnitt (12) des Körpers (2) längsgerichtet zwischen 5,0 mm bis 10,0 mm von der Spitze (3) befindet.

10. Nasenstück nach einem vorstehenden Anspruch, wobei eine Längsachse des ersten aufgeweiteten Abschnitts (10) mit einer Längsachse des zweiten aufgeweiteten Abschnitts (11) zusammenfällt.

11. Nasenstück nach einem vorstehenden Anspruch, wobei der Körper (2) weiter eine oder mehrere Schultern (15) zum Eingriff durch einen oder mehrere Finger umfasst, wobei sich die eine oder mehreren Schultern (15) weiter von der Spitze (3) befinden als der zweite aufgeweitete Abschnitt (11).

12. Nasenspray, Nasenkanüle, Vernebler oder Nasendilatator, umfassend ein Nasenstück nach einem vorstehenden Anspruch.

13. Verwendung eines Nasenstücks nach einem der Ansprüche 1 bis 11, wobei in einer ersten Verwendungsweise das Nasenstück in ein erstes Nasenloch mit einem ersten Größenbereich derart eingeführt wird, dass das erste Nasenloch an der ersten Anstoßzone (20) des ersten aufgeweiteten Abschnitts (10) anstößt; und in einer zweiten Verwendungsweise das Nasenstück in ein zweites Nasenloch mit einem zweiten Größenbereich derart eingeführt wird, dass das zweite Nasenloch gegen die zweite Anstoßzone (21) des zweiten aufgeweiteten Abschnitts (11) anstößt;
wobei das erste Nasenloch optional ein Nasenloch eines pädiatrischen Benutzers ist und das zweite Nasenloch ein Nasenloch eines erwachsenen Benutzers ist.

14. Verfahren zum Herstellen eines Nasenstücks nach einem der Ansprüche 1 bis 11, umfassend das Bilden des Körpers (2) des Nasenstücks, um die Spitze (3), den ersten aufgeweiteten Abschnitt (10), der sich in einer Längsrichtung von der Spitze (3) weg erweitert, und den zweiten aufgeweiteten Abschnitt (11) aufzuweisen, der sich ebenfalls in der Längsrichtung von der Spitze (3) weg erweitert, wobei der erste aufgeweitete Abschnitt (10) näher an der Spitze vorliegt als der zweite aufgeweitete Abschnitt (11), und der erste aufgeweitete Abschnitt (10) die erste Anstoßzone (20) zum Eingreifen in das erste Nasenloch mit dem ersten Größenbereich definiert und der zweite aufgeweitete Abschnitt (11) die zweite Anstoßzone (21) zum Eingreifen in das zweite Nasenloch mit dem zweiten Größenbereich definiert, wobei die erste Anstoßzone (20) einen maximalen Aufweitungswinkel aufweist, der größer ist als ein maximaler Aufweitungswinkel der zweiten Anstoßzone (21);
wobei das Nasenstück optional als ein einzelnes Stück gebildet ist; und optional durch Formung, vorzugsweise durch Spritzgießen, gebildet wird.

## Revendications

1. Embout nasal (1) comprenant un corps (2) muni d'une pointe (3) destiné à être inséré dans une narine présentant une plage de tailles, le corps (2) comprenant :
une première section évasée (10) qui s'élargit dans une direction longitudinale à partir de la pointe (3) ;
une deuxième section évasée (11) qui s'élargit également dans la direction longitudinale à partir de la pointe (3) ;
la première section évasée (10) étant plus proche de la pointe (3) que la deuxième section évasée (11) ;
la première section évasée (10) définissant une première zone de butée (20) pour se mettre en prise dans une première narine présentant une première plage de tailles ;
la deuxième section évasée (11) définissant une deuxième zone de butée (21) pour se mettre en prise dans une deuxième narine présentant une deuxième plage de tailles ;
dans lequel la première zone de butée (20) présente un angle d'évasement maximal supérieur à l'angle d'évasement maximal de la deuxième zone de butée (21) ;
dans lequel le corps (2) comprend un épaulement ou une section convexe (12) qui sépare au moins une partie de la première zone de butée (20) d'au moins une partie de la deuxième zone de butée (21) ;
dans lequel la première section évasée (10) comprend une section concave et la deuxième section évasée (11) comprend une section concave ; et
dans lequel le corps (2) comprend en outre une section de pointe (13) qui comprend la pointe (3) du corps (2) et s'étend de la pointe (3) à la première section évasée (10), dans lequel la section de pointe (13) est convexe.

2. Embout nasal selon la revendication 1, dans lequel l'angle d'évasement maximal de la première zone de butée (20) est de 35° à 65°, de préférence de 40° à 60°, et plus préférentiellement de 58°, et/ou l'angle d'évasement maximal de la deuxième zone de butée (21) est de 10° à 30°, de préférence de 15° à 25°, plus préférentiellement de 18°.

3. Embout nasal selon la revendication 1 ou la revendication 2, dans lequel, soit :
un angle d'évasement de la première zone de butée (20) est constant sur toute la longueur longitudinale de la première zone de butée (20) ; et éventuellement, dans lequel l'angle d'évasement de la première zone de butée (20) est de 35° à 65°, de préférence de 40° à 60°, plus préférentiellement de 58° ;
soit
un angle d'évasement de la première zone de butée (20) varie le long d'une longueur longitudinale de la première zone de butée (20) ; et éventuellement, dans lequel l'angle d'évasement varie d'un angle d'évasement minimum de 15° à 20° à un angle d'évasement maximum de 35° à 65°, de préférence de 40° à 60°, plus préférentiellement de 58°.

4. Embout nasal tel selon une quelconque revendication précédente, dans lequel soit :
un angle d'évasement de la deuxième zone de butée (21) est constant sur toute la longueur longitudinale de la deuxième zone de butée (21) ; et éventuellement, dans lequel l'angle d'évasement de la deuxième zone de butée (21) est de 10° à 30°, de préférence de 15° à 25°, plus préférentiellement de 18° ;
soit
un angle d'évasement de la deuxième zone de butée (21) varie le long d'une longueur longitudinale de la deuxième zone de butée (21) ; et éventuellement, dans lequel l'angle d'évasement varie d'un angle d'évasement minimum de 5° à 10° à un angle d'évasement maximum de 10° à 30°, de préférence de 15° à 25°, plus préférentiellement de 18°.

5. Embout nasal selon une quelconque revendication précédente, dans lequel une longueur longitudinale de la première zone de butée (20) et/ou la première section évasée (10) est plus courte qu'une longueur longitudinale de la deuxième zone de butée (21) et/ou la deuxième section évasée (11).

6. Embout nasal selon une quelconque revendication précédente, dans lequel :
la première zone de butée (20) s'étend au moins entre des emplacements situés à 5,0 mm à 7,0 mm longitudinalement de la pointe (3) ; et/ou
la deuxième zone de butée (21) s'étend au moins entre des emplacements situés à 12,0 mm à 15,0 mm longitudinalement de la pointe (3) ; éventuellement au moins entre des emplacements situés à 12,0 mm à 20,0 mm longitudinalement de la pointe (3) ; éventuellement au moins entre des emplacements situés à 10,0 mm à 20,0 mm longitudinalement de la pointe (3).

7. Embout nasal selon une quelconque revendication précédente, dans lequel la deuxième section évasée (11) est plus large que la première section évasée (10).

8. Embout nasal selon une quelconque revendication précédente, dans lequel :
un diamètre extérieur de la première zone de butée (20) à son extrémité la plus proche de la pointe (3) présente un diamètre de 6,60 à 7,50 mm, éventuellement de 6,90 à 7,20 mm, éventuellement de 7,09 mm ; et/ou un diamètre extérieur de la première zone de butée (20) à son extrémité la plus éloignée de la pointe (3) présente un diamètre de 9,50 à 11,00 mm, éventuellement de 9,75 à 10,50 mm, éventuellement de 10,43 mm ; et/ou
un diamètre extérieur de la deuxième zone de butée (21) à son extrémité la plus proche de la pointe (3) présente un diamètre de 10,50 à 11,50 mm, éventuellement de 10,70 à 11,00 mm, éventuellement de 10,70 à 10,90 mm, éventuellement de 10,85 mm ou 10,96 mm ; et/ou un diamètre extérieur de la deuxième zone de butée (21) à son extrémité la plus éloignée de la pointe (3) présente un diamètre de 11,00 à 20,00 mm, éventuellement de 11,00 à 16,00 mm, éventuellement de 11,00 à 15,00 mm, éventuellement de 11,75 à 15,00 mm, éventuellement de 14,00 à 15,00 mm, éventuellement de 11,75 mm ou 14,75 mm.

9. Embout nasal selon une quelconque revendication précédente, dans lequel l'épaulement ou la section convexe (12) du corps (2) est située entre 5,0 mm et 10,0 mm longitudinalement de la pointe (3).

10. Embout nasal selon une quelconque revendication précédente, dans lequel un axe longitudinal de la première section évasée (10) coïncide avec un axe longitudinal de la deuxième section évasée (11).

11. Embout nasal selon une quelconque revendication précédente, dans lequel le corps (2) comprend en outre un ou plusieurs épaulements (15) destinés à être mis en prise par un ou plusieurs doigts, les un ou plusieurs épaulements (15) étant situés plus loin de la pointe (3) que la deuxième section évasée (11).

12. Spray nasal, canule nasale, nébuliseur ou dilatateur nasal comprenant un embout nasal selon une quelconque revendication précédente.

13. Utilisation d'un embout nasal selon l'une quelconque des revendications 1 à 11, dans lequel, dans un premier mode d'utilisation, l'embout nasal est inséré dans une première narine présentant une première plage de tailles, de telle sorte que la première narine soit en butée contre la première zone de butée (20) de la première section évasée (10) ; et dans un deuxième mode d'utilisation, l'embout nasal est inséré dans une deuxième narine présentant une deuxième plage de tailles de telle sorte que la deuxième narine soit en butée contre la deuxième zone de butée (21) de la deuxième section évasée (11) ;
dans lequel, éventuellement, la première narine est celle d'un utilisateur pédiatrique et la deuxième narine celle d'un utilisateur adulte.

14. Procédé de fabrication d'un embout nasal selon l'une quelconque des revendications 1 à 11, comprenant la formation du corps (2) de l'embout nasal de manière à ce que la pointe (3), la première section évasée (10) s'élargisse dans une direction longitudinale à partir de la pointe (3) et la deuxième section évasée (11) s'élargisse également dans une direction longitudinale à partir de la pointe (3), dans lequel la première section évasée (10) est plus proche de la pointe que la deuxième section évasée (11), et la première section évasée (10) définit la première zone de butée (20) pour se mettre en prise dans la première narine présentant la première plage de tailles, et la deuxième section évasée (11) définit la deuxième zone de butée (21) pour se mettre en prise dans la deuxième narine présentant la deuxième plage de tailles, dans lequel la première zone de butée (20) présente un angle d'évasement maximal supérieur à l'angle d'évasement maximal de la deuxième zone de butée (21) ;
dans lequel, éventuellement, l'embout nasal est formé d'une seule pièce ; et, éventuellement, formée par moulage, de préférence par moulage par injection.
